(19)

Europäisches
Patentamt
European
Patent Office
Office européen
des brevets

(11) **EP 4 086 356 A1**

(12) **EUROPEAN PATENT APPLICATION**
published in accordance with Art. 153(4) EPC

(43) Date of publication:
**09.11.2022 Bulletin 2022/45**

(21) Application number: **19958118.2**

(22) Date of filing: **31.12.2019**

(51) International Patent Classification (IPC):
*C12Q 1/68* (2018.01)          *G16B 20/10* (2019.01)
*G16B 25/10* (2019.01)

(86) International application number:
**PCT/CN2019/130625**

(87) International publication number:
**WO 2021/134513 (08.07.2021 Gazette 2021/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **BGI Clinical Laboratories (Shenzhen)
Co., Ltd.
Shenzhen, Guangdong 518083 (CN)**

(72) Inventors:
• **ZHANG, Hongyun
  Shenzhen, Guangdong 518083 (CN)**

• **YUAN, Yuying
  Shenzhen, Guangdong 518083 (CN)**
• **CHAI, Xianghua
  Shenzhen, Guangdong 518083 (CN)**
• **ZHOU, Lijun
  Shenzhen, Guangdong 518083 (CN)**
• **WANG, Mengjie
  Shenzhen, Guangdong 518083 (CN)**
• **LIU, Qiang
  Shenzhen, Guangdong 518083 (CN)**
• **YIN, Ye
  Shenzhen, Guangdong 518083 (CN)**

(74) Representative: **Hoffmann Eitle
Patent- und Rechtsanwälte PartmbB
Arabellastraße 30
81925 München (DE)**

(54) **METHODS FOR DETERMINING CHROMOSOME ANEUPLOIDY AND CONSTRUCTING
CLASSIFICATION MODEL, AND DEVICE**

(57)     Provided is a method for determining whether a fetus has chromosomal aneuploidy, the method comprising: the method comprises: (1) obtaining nucleic acid sequencing data from a pregnant woman sample; (2) determining a fetal fraction of the pregnant woman sample and an estimated fraction by a predetermined chromosome based on the nucleic acid sequencing data; (3) determining a first feature based on a difference between the estimated fraction by a chromosome to be tested and the estimated fraction by a second comparison chromosome, and determining a second feature based on a difference between the estimated fraction by the chromosome to be tested and the fetal fraction; and (4) determining whether the fetus has an aneuploidy for the chromosome to be tested based on the first feature and the second feature by using corresponding data of control sample, wherein the control sample comprises a positive sample and a negative sample, the positive sample has an aneuploidy for the chromosome to be tested, and the negative sample does not have an aneuploidy for the chromosome to be tested.

EP 4 086 356 A1

## Description

**Technical Field**

The present invention relates to the field of biotechnology, especially non-invasive prenatal genetic testing, and specifically relates to a method and device for determining chromosomal aneuploidy and a corresponding method and device for constructing a machine learning classification model.

**Background Art**

Prenatal screening methods are usually divided into two categories, namely invasive methods (also called prenatal diagnosis) and non-invasive methods. The former mainly include amniocentesis, villus sampling, cord blood sampling, etc.; the latter include ultrasonography, maternal peripheral serum marker determination, fetal cell detection, etc. The invasive methods such as chorionic villus sampling (CVS) or amniocentesis are used to obtain cells isolated from fetuses, which can be used for routine prenatal diagnosis. Although these methods are highly accurate in diagnosing fetal aneuploidy, these conventional methods are invasive and have certain risks for both pregnant women and fetuses.
Conventional non-invasive screening methods, such as prenatal serological screening, are usually less accurate.
Dennis Lo, et al. find that there is cell-free fetal DNA in maternal plasma and serum, thereby providing a new idea for non-invasive prenatal testing (NIPT). Non-invasive prenatal testing mainly uses high-throughput sequencing technology to analyze the cell-free fetal DNAs in the peripheral blood of pregnant women to assess the risk of common chromosomal aneuploidy in the fetus. At present, the common screening scopes are chromosome 21 aneuploidy (T21), chromosome 18 aneuploidy (T18), chromosome 13 aneuploidy (T13) and sex chromosomes.
Based on high-throughput sequencing technology, the existing common technologies for detecting fetal chromosomal aneuploidy using cell-free fetal DNAs in the peripheral blood of pregnant women are as follows:

1. NIPT performed by the method based on quantification for number of reads: The main principle of this method is that reads (sometimes called "sequencing reads") are located in pre-defined windows by using a comparison software, and then an appropriate method is used to perform the aneuploidy detection of the chromosome to be tested.

2. NIPT performed by the method based on single nucleotide polymorphism (SNP): The main principle of this method is that to the acquisition and sequencing of the genomic DNAs of both parents and the cell-free fetal DNAs are respectively performed based on the predetermined SNP site regions, and then the aneuploidy detection of the chromosome to be tested is performed by the Bayesian model using the genotype information of the parents and the fetus.

3. NIPT performed by the method based on size of DNA fragments: The main idea of this method is that the cell-free fetal DNA fragments are specifically extracted by using paired-end (PE) sequencing technology based on the distribution difference between the cell-free fetal DNA fragments and the maternal DNA fragments, and finally the aneuploidy detection of the chromosome to be tested is performed by using the Z-test based on the reference samples.

However, these existing non-invasive prenatal diagnosis methods each have their own shortcomings. In order to facilitate understanding, they are summarized in the following table:

| Technology | Disadvantages |
| --- | --- |
| NIPT performed by the method based on quantification for number of reads | Requirement for setting threshold, resulting in the introduction of detection gray area. |
| NIPT performed by the method based on single nucleotide polymorphism (SNP) | High cost, limitation of detection area caused by the selected SNP site, and requirement for acquisition and analysis of haplotype information of both parents |
| NIPT performed by the method based on size of DNA fragments | Requirement of using PE sequencing, high cost |

Therefore, the current methods for determining chromosomal aneuploidy through non-invasive methods still need to be improved.

## Contents of the Invention

The present invention aims to solve at least one of the technical problems existing in the prior art. For this reason, an object of the present invention is to provide a method that can effectively determine chromosomal aneuploidy.

According to one aspect of the present invention, the present invention provides a method for determining whether a fetus has a chromosomal aneuploidy. According to an embodiment of the present invention, the method comprises: (1) acquiring nucleic acid sequencing data from a pregnant woman sample, wherein the pregnant woman sample comprises a cell-free fetal nucleic acid, and the nucleic acid sequencing data are composed of a plurality of sequencing reads; (2) determining a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; (3) determining a first feature based on a difference between the estimated fraction by the chromosome to be tested and the estimated fraction by the second comparison chromosome, and determining a second feature based on a difference between the estimated fraction by the chromosome to be tested and the fetal fraction; and (4) determining whether the fetus has an aneuploidy for the chromosome to be tested based on the first feature and the second feature by using corresponding data of a control sample, wherein the control sample comprises a positive sample and a negative sample, the positive sample has aneuploidy for the chromosome to be tested, and the negative sample does not have aneuploidy for the chromosome to be tested.

This method can effectively determine whether the fetus has an aneuploidy for the chromosome to be tested. In addition, according to the embodiment of the present invention, in the process of implementing the method, it is found in the method that the strategy of setting threshold based on the number of sequencing reads in the prior art is replaced, the detection gray area is avoided, the sample detection cycle is also shortened, and thus the customer experience is improved, and the sequencing and testing costs are significantly reduced.

According to an embodiment of the present invention, the above method may also have an additional technical feature as follows:

According to an embodiment of the present invention, the pregnant woman sample comprises peripheral blood of a pregnant woman.

According to an embodiment of the present invention, the nucleic acid sequencing data are obtained by paired-end sequencing, single-end sequencing, or single-molecule sequencing.

According to an embodiment of the present invention, the fetal fraction is determined by the following steps: (a) comparing the nucleic acid sequencing data from the pregnant woman sample with a reference sequence, so as to determine the number of sequencing reads that fall into a predetermined window; and (b) determining the fetal fraction of the pregnant woman sample based on the number of sequencing reads that fall into the predetermined window.

According to an embodiment of the present invention, in the step (2), the number of sequencing reads of the first comparison chromosome is an average number of sequencing reads of a plurality of autosomes, and the plurality of autosomes comprise at least one autosome which is not known to have aneuploidy.

According to an embodiment of the present invention, in the step (2), the number of sequencing reads of the first comparison chromosome is an average number of sequencing reads of at least 15 autosomes; optionally, the number of sequencing reads of the first comparison chromosome is an average number of sequencing reads of at least 20 autosomes; optionally, the number of sequencing reads of the first comparison chromosome is an average number of sequencing reads of all autosomes.

According to an embodiment of the present invention, the estimated fraction is determined according to the following formula:

$$F_j = 2 \times \left| R_j - R_r \right| / R_r$$

wherein,

j represents the serial number of the chromosome the estimated fraction of which needs to be determined,
$F_j$ represents the estimated fraction by the chromosome $j$,
$R_r$ represents the average number of sequencing reads of the plurality of autosomes, and
$R_j$ represents the number of sequencing reads of the chromosome $j$.

**EP 4 086 356 A1**

According to an embodiment of the present invention, in the step (3), the first feature is determined based on the difference between the estimated fraction by the chromosome to be tested and an average value of the estimated fraction by the second comparison chromosome.

According to an embodiment of the present invention, the second comparison chromosome comprises at least 10 autosomes.

According to an embodiment of the present invention, the second comparison chromosome comprises 15 autosomes.

According to an embodiment of the present invention, the method further comprises: determining the estimated fraction by a plurality of autosomes; and selecting a target autosome as the second comparison chromosome in a priority order from small to large.

According to an embodiment of the present invention, the first feature is determined by the following formula:

$$X_1 = F_i - F_r$$

wherein,

$X_1$ represents the first feature,
$i$ represents the serial number of the chromosome to be tested,
$F_i$ represents the estimated fraction by the chromosome to be tested,
$F_r$ represents the average value of the estimated fraction by the second comparison chromosome.

According to an embodiment of the present invention, the second feature is determined by the following formula:

$$X_2 = \frac{F_i}{F_a}$$

wherein,

$X_2$ represents the second feature,
$i$ represents the serial number of the chromosome to be tested,
$F_i$ represents the estimated fraction by the chromosome to be tested,
$F_a$ represents the fetal fraction.

According to an embodiment of the present invention, before performing the step (4), the first feature and the second feature are standardized, so that the absolute values of the first feature and the second feature are independently between 0 to 1.

According to an embodiment of the present invention, in the step (4), the numbers of the positive sample and the negative sample have a ratio of not less than 1:4.

According to an embodiment of the present invention, in the step (4), the numbers of the positive sample and the negative sample have a ratio of not exceeding 4:1.

According to an embodiment of the present invention, in the step (4), the numbers of the positive sample and the negative sample have a ratio of 1:0.1-5.

According to an embodiment of the present invention, in the step (4), the numbers of the positive sample and the negative sample have a ratio of 1:0.25~4.

According to an embodiment of the present invention, neither the positive sample nor the negative sample has aneuploidy for chromosomes other than the chromosome to be tested.

According to an embodiment of the present invention, in the step (4), the first feature and the second feature are used to determine a two-dimensional feature vector of the pregnant woman sample and the control samples, a distance between samples is determined based on the two-dimensional feature vector, the pregnant woman sample is classified as positive sample or negative sample, so as to determine whether the fetus has an aneuploidy for the chromosome to be tested.

According to an embodiment of the present invention, the distance is an Euclidean distance, a Manhattan distance or a Chebyshev distance.

According to an embodiment of the present invention, the step (4) further comprises: (4-1) calculating distances between the pregnant woman sample and the control samples respectively; (4-2) sorting the obtained distances, the sorting being based on the order from small to large; (4-3) selecting a predetermined number of control samples in order from small to large based on the sorting; (4-4) determining the number of positive samples and the number of negative samples in the predetermined number of control samples respectively; (4-5) determining a classification result of the pregnant woman sample based on a majority decision method.

According to an embodiment of the present invention, the predetermined number is not more than 20.

According to an embodiment of the present invention, the predetermined number is 3 to 10.

According to an embodiment of the present invention, in the step (4-2), before the sorting is performed, the distances between the sample to be tested and the predetermined control samples are weighted in advance.

In the second aspect of the present invention, the present invention provides a device for determining whether a fetus has a chromosomal aneuploidy, which is characterized by comprising: a data acquisition module, which is configured to acquire nucleic acid sequencing data from a pregnant woman sample, wherein the pregnant woman sample comprises a fetal free nucleic acid, and the nucleic acid sequencing data are composed of a plurality of sequencing reads; a fetal fraction-estimated fraction determination module, which is configured to determine a fetal fraction of the pregnant woman sample and the estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; a feature determination module, which is configured to determine a first feature based on a difference between the estimated fraction by the chromosome to be tested and the estimated fraction by the second comparison chromosome, and to determine a second feature based on a difference between the estimated fraction by the chromosome to be tested and the fetal fraction; and an aneuploidy determination module, which is configured to determine whether the fetus of the pregnant woman has an aneuploidy for the chromosome to be tested based on the first feature and the second feature by using corresponding data of a control sample, wherein the control sample comprises a positive sample and a negative sample, the positive sample has an aneuploidy for the chromosome to be tested, and the negative sample does not have an aneuploidy for the chromosome to be tested. By using the device for determining whether a fetus has a chromosomal aneuploidy according to an embodiment of the present invention, the forgoing method for determining whether a fetus has a chromosomal aneuploidy can be effectively implemented, and thus it can be effectively determined whether the fetus has an aneuploidy for the chromosome to be tested. In addition, according to an embodiment of the present invention, in the process of implementing the method of the present invention, it is found in this method that the strategy of setting threshold based on the number of sequencing reads in the prior art is replaced, the detection gray area is avoided, the sample detection cycle is also shortened, and thus the customer experience is improved, and the sequencing and testing costs are significantly reduced.

According to an embodiment of the present invention, the device may also have an additional technical feature as follows:

According to an embodiment of the present invention, the fetal fraction-estimated fraction determination module comprises: an alignment unit, which is configured to align the nucleic acid sequencing data from the pregnant woman sample with a reference sequence, so as to determine the number of sequencing reads that fall into a predetermined window; and a fetal fraction calculation unit, which is configured to determine the fetal fraction of the pregnant woman sample based on the number of sequencing reads that fall into the predetermined window.

According to an embodiment of the present invention, the fetal fraction-estimated fraction determination module comprises: an estimated fraction calculation unit, which is configured to determine the estimated fraction according to the following formula:

$$F_j = 2 \times \left| R_j - R_r \right| / R_r$$

wherein,

$j$ represents the serial number of the chromosome the estimated fraction of which needs to be determined,

$F_j$ represents the estimated fraction by the chromosome $j$,

$R_r$ represents the average number of sequencing reads of the plurality of autosomes, and

$R_j$ represents the number of sequencing reads of the chromosome $j$.

According to an embodiment of the present invention, the fetal fraction-estimated fraction determination module comprises: a second comparison chromosome determination unit, which is configured to sort the estimated fraction by a plurality of autosomes in a priority order from small to large, and select target autosomes from the sorted autosomes as the second comparison chromosome.

According to an embodiment of the present invention, the feature determination module comprises:
a first feature determination unit, which is configured to determine the first feature by the following formula:

$$X_1 = F_i - F_r$$

wherein,

$X_1$ represents the first feature,
$i$ represents the serial number of the chromosome to be tested,
$F_i$ represents the estimated fraction by the chromosome to be tested,
$F_r$ represents the average value of the estimated fraction by the second comparison chromosome.

According to an embodiment of the present invention, the feature determination module comprises: a second feature determination unit, which is configured to determine the second feature by the following formula:

$$X_2 = \frac{F_i}{F_a}$$

wherein,

$X_2$ represents the second feature,
$i$ represents the serial number of the chromosome to be tested,
$F_i$ represents the estimated fraction by the chromosome to be tested,
$F_a$ represents the fetal fraction.

According to an embodiment of the present invention, the feature determination module comprises: a standardization processing unit, which is configured to perform standardization on the first feature and the second feature, so that the absolute values of the first feature and the second feature are independently between 0 to 1.

According to an embodiment of the present invention, the aneuploidy determination module is configured to determine a two-dimensional feature vector of the pregnant woman sample and the control samples, a distance between samples is determined based on the two-dimensional feature vector, and the pregnant woman sample is classified as positive sample or negative sample, so as to determine whether the fetus has an aneuploidy for the chromosome to be tested.

According to an embodiment of the present invention, the distance is an Euclidean distance, a Manhattan distance or a Chebyshev distance.

According to an embodiment of the present invention, the aneuploidy determination module is configured to determine a classification result of the pregnant woman sample by using a $k$-nearest neighbor model.

According to an embodiment of the present invention, the $k$-nearest neighbor model adopts a $k$ value of not exceeding 20.

According to an embodiment of the present invention, the $k$-nearest neighbor model adopts a $k$ value of 3 to 10.

According to an embodiment of the present invention, in the $k$-nearest neighbor model, the distance between samples is weighted.

In the third aspect of the present invention, the present invention provides a computer-readable storage medium, on which a computer program is stored, characterized in that, when the program is executed by a processor, the steps of the aforementioned method for determining whether a fetus has a chromosomal aneuploidy are implemented. Therefore, the aforementioned method for determining whether a fetus has a chromosomal aneuploidy can be effectively implemented, so that it can be effectively determined whether the fetus has an aneuploidy for the chromosome to be tested. In addition, according to an embodiment of the present invention, in the process of implementing the method, it is found in the method that the strategy of setting threshold based on the number of sequencing reads in the prior art is replaced, the detection gray area is avoided, the sample detection cycle is also shortened, and thus the customer experience is improved, and the sequencing and testing costs are significantly reduced.

In the fourth aspect of the present invention, the present invention provides an electronic device, which comprises: the aforementioned computer-readable storage medium; and one or more processors, which are configured to execute the program stored on the computer-readable storage medium. Therefore, the aforementioned method for determining whether a fetus has a chromosomal aneuploidy can be effectively implemented, so that it can be effectively determined whether the fetus has an aneuploidy for the chromosome to be tested. In addition, according to an embodiment of the present invention, in the process of implementing the method, it is found in the method that the strategy of setting threshold based on the number of sequencing reads in the prior art is replaced, the detection gray area is avoided, the sample detection cycle is also shortened, and thus the customer experience is improved, and the sequencing and testing costs are significantly reduced.

In the fifth aspect of the present invention, the present invention provides a method for constructing a machine learning classification model. According to an embodiment of the present invention, the method comprises: (a) performing the following steps for each of a plurality of pregnant women samples: acquiring nucleic acid sequencing data from the pregnant woman sample, wherein the pregnant woman sample comprise a fetal free nucleic acid, the nucleic acid sequencing data are composed of a plurality of sequencing reads, the pregnant woman sample comprises at least one positive sample and at least one negative sample, the positive sample has an aneuploidy for a chromosome to be tested, and the negative sample does not have an aneuploidy for the chromosome to be tested; determining a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; and determining a first feature based on a difference between the estimated fraction by the chromosome to be tested and the estimated fraction by the second comparison chromosome, and determining a second feature based on a difference between the estimated fraction by the chromosome to be tested and the fetal fraction; (b) performing a machine learning training by taking the plurality of pregnant women samples as samples and using the first features and the second features of the samples, so as to construct a machine learning classification model for determining whether the fetus has an aneuploidy. By using this method, according to an embodiment of the present invention, a machine learning classification model can be effectively constructed, so that the classification model can be further used to identify and classify unknown samples to determine whether there is a chromosomal aneuploidy for a specific chromosome.

According to an embodiment of the present invention, the machine learning classification model is $k$-nearest neighbors (KNN) model.

According to an embodiment of the present invention, the KNN model adopts an Euclidean distance.

In the sixth aspect of the present invention, the present invention provides a device for constructing a machine learning classification model, which comprises: a feature acquisition module, which is configured to perform the following steps for each of a plurality of pregnant women samples: acquiring nucleic acid sequencing data from the pregnant woman sample, wherein the pregnant woman sample comprise a fetal free nucleic acid, the nucleic acid sequencing data are composed of a plurality of sequencing reads, the pregnant woman sample comprises at least one positive sample and at least one negative sample, the positive sample has an aneuploidy for a chromosome to be tested, and the negative sample does not have an aneuploidy for the chromosome to be tested; determining a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; and determining a first feature based on a difference between the estimated fraction by the chromosome to be tested and the estimated fraction by the second comparison chromosome, and determining a second feature based on a difference between the estimated fraction by the chromosome to be tested and the fetal fraction; a training model, which is configured to perform a machine learning training by taking the plurality of pregnant women samples as samples, so as to construct a machine learning classification model for determining whether the fetus has an aneuploidy. By using this device, the aforementioned method for constructing a machine learning classification model can be effectively implemented, thereby effectively constructing a machine learning classification model, so that the classification model can be further used to identify and classify unknown samples to determine whether there is a chromosomal aneuploidy for a specific chromosome.

According to an embodiment of the present invention, the machine learning classification model is a KNN model.

In the seventh aspect of the present invention, the present invention provides a computer-readable storage medium, on which a computer program is stored, and when the program is executed by a processor, the steps for constructing a machine learning classification method described in the preceding claims are implemented. As a result, the aforementioned method for constructing a machine learning classification model can be effectively implemented, thereby effectively constructing a machine learning classification model, so that the classification model can be further used to identify and classify unknown samples to determine whether there is a chromosomal aneuploidy for a specific chromosome.

The additional aspects and advantages of the present invention will be partly given in the following description, and partly will become obvious from the following description, or be understood through the practice of the present invention.

**Brief Description of the Drawings**

The above and/or additional aspects and advantages of the present invention will become obvious and easy to understand from the description of the embodiments in conjunction with the following drawings, in which:

Figure 1 shows a schematic flow chart of a method for determining whether a fetus has chromosomal aneuploidy according to an embodiment of the present invention;

Figure 2 shows a schematic flow chart of a method for determining fetal fraction according to an embodiment of the present invention;

Figure 3 shows a schematic flow chart of a method for classifying pregnant women samples according to an embodiment of the present invention;

Figure 4 shows a block diagram of an apparatus for determining whether a fetus has chromosomal aneuploidy according to an embodiment of the present invention;

Figure 5 shows a block diagram of a fetal fraction-estimated fraction determination module according to an embodiment of the present invention;

Figure 6 shows a block diagram of a feature determination module according to an embodiment of the present invention;

Figure 7 shows a block diagram of constructing a machine learning classification model according to an embodiment of the present invention;

Figures 8 and 9 show the ROC curves corresponding to the parameter k when using the KNN model to detect T21 according to an embodiment of the present invention;

Figures 10 and 11 show the ROC curves corresponding to the parameter k when using the KNN model to detect T 18 according to an embodiment of the present invention; and

Figures 12 and 13 show the ROC curves corresponding to the parameter k when using the KNN model to detect T 13 according to an embodiment of the present invention.

**Specific Models for Carrying Out the Invention**

The embodiments of the present invention are described in detail below. The embodiments described below are exemplary, and are only used to explain the present invention, but should not be construed as limiting the present invention. It should be noted that this application can be used in many general or special computing device environments or configurations. For example: personal computers, server computers, handheld or portable devices, tablet devices, multi-processor devices, distributed computing environments comprising any of the above apparatus or devices, and the like. This application may be described in the general context of computer-executable instructions executed by a computer, such as program modules. Generally, program modules comprise routines, programs, objects, components, data structures, etc. that perform specific tasks or implement specific abstract data types. This application can also be practiced in distributed computing environments. In these distributed computing environments, tasks are performed by remote processing devices connected through a communication network. In such distributed computing environments, program modules can be stored in local and remote computer storage media including storage devices.

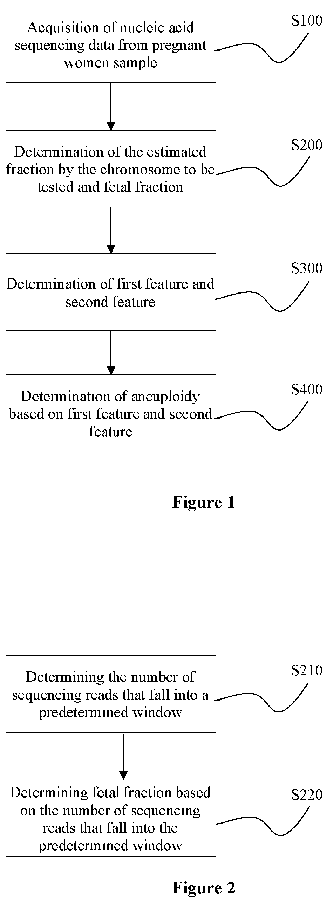

According to one aspect of the present invention, the present invention provides a method for determining whether a fetus has a chromosomal aneuploidy. The method for determining whether a fetus has a chromosomal aneuploidy according to an embodiment of the present invention is described in detail below by referring to Figures 1 to 3.

Referring to Figure 1, according to an embodiment of the present invention, the method for determining whether a fetus has a chromosomal aneuploidy comprises:

**S100: Acquisition of nucleic acid sequencing data from pregnant women sample**

According to an embodiment of the present invention, in this step, nucleic acid sequencing data are firstly acquired from a pregnant woman sample that contains a fetal free nucleic acid. For example, according to an embodiment of the present invention, the pregnant woman sample that can be used includes but is not limited to a peripheral blood of a pregnant woman. As mentioned earlier, Dennis Lo et al. has found that there is non-cellular free fetal DNA in maternal plasma and serum, thereby providing a new idea for non-invasive prenatal testing (NIPT). By using the peripheral blood of the pregnant woman, it would not cause trauma to the pregnant woman and avoid the risk of miscarriage due to sampling. According to an embodiment of the present invention, when collecting a pregnant women sample, such as a peripheral blood of pregnant women, nucleic acid sequencing can be performed on such sample to obtain nucleic acid sequencing data of the pregnant women sample. Generally, the nucleic acid sequencing data are composed of a plurality of or a large number of sequencing reads. According to an embodiment of the present invention, the method for sequencing nucleic acid molecules of the pregnant women sample is not particularly limited. Specifically, any sequencing method known to those skilled in the art, for example, including but not limited to paired-end sequencing, single-end sequencing, end-sequencing or single-molecule sequencing, can be used to sequence the nucleic acid molecules of the pregnant women sample.

Those skilled in the art can understand that after acquiring the nucleic acid sequencing data, the acquired sequencing data composed of a large number of sequencing reads can be filtered and screened according to quality control standards so as to remove sequencing reads with sequencing quality problems, which can improve the accuracy of subsequent data analysis.

**S200: Determination of the estimated fraction and fetal fraction**

After acquiring the nucleic acid sequencing data from the pregnant woman sample, by analyzing the number of sequencing reads of the nucleic acid sequencing data, a fetal fraction of the pregnant woman sample and the estimated fraction by a specific chromosome can be determined.

According to an embodiment of the present invention, the fetal fraction refers to a ratio of the number of free nucleic acids from a fetus to the number of total free nucleic acids in the free nucleic acids of a pregnant women sample, such as peripheral blood. Usually, the value of the fetal fraction will increase with the increase of gestational weeks. For example, around the 12th gestational week, the ratio of the fetal free nucleic acids (sometimes directly referred to as "fetal free DNA") to the total free nucleic acid (i.e., "fetal fraction") can reach 10-14%, and after the 20th gestational week, this ratio can reach more than 20%. When the fetus has an abnormal condition, such as chromosomal aneuploidy, the fetal fraction will be abnormal. Therefore, the fetal fraction can be used as an important indicator to characterize the pregnant women sample.

Those skilled in the art can obtain fetal fraction data of a pregnant woman sample by various known methods. For example, according to an embodiment of the present invention, methods including but not limited to Y chromosome estimation method, SNP-based fetal-specific SNP site method, and nucleosome-based imprinting method can be used. However, the inventors of the present invention found that these methods have their limitations. For example, the Y chromosome estimation method is not suitable for female fetuses, the SNP-based fetal-specific SNP site method needs to obtain DNA samples of father (sometimes these samples can hardly be obtained), and the nucleosome-based imprinting method has poor accuracy and needs to perform deep sequencing when constructing model.

Referring to Figure 2, according to an embodiment of the present invention, the fetal fraction of a nucleic acid sample can be determined through the following steps, which specifically comprise:

S210: comparing the nucleic acid sequencing data from a pregnant woman sample with a reference sequence to determine the number of sequencing reads that fall into a predetermined window; and

S220: determining the fetal fraction of the pregnant woman sample based on the number of sequencing reads that fall into the predetermined window.

The method for determining fetal fraction is based on the number of sequencing reads in a specific window (i.e., a certain length of nucleic acid sequence), which is positively correlated with fetal fraction. Therefore, by determining the number of sequencing reads in at least one predetermined window, the fetal fraction of the pregnant woman sample can be inversely obtained, for example, in a weighted average manner. The predetermined window can be determined by means of statistics or machine learning. According to an embodiment of the present invention, the predetermined window is obtained by continuous dividing of specific chromosomes of a reference genome sequence, and the weight of each predetermined window is further used to determine the fetal fraction. According to some specific examples of the present invention, the weight of each predetermined window is predetermined by using training samples. Therefore, the results are accurate, reliable, and repeatable.

According to an embodiment of the present invention, the weight is determined using at least one of a ridge regression statistical model and a neural network model. According to some embodiments of the present invention, the neural network model adopts a TensorFlow learning system. According to some specific examples of the present invention, the parameters of the TensorFlow learning system include: adopting the number of sequencing data of autosomes in each window as the input layer; adopting the fetal fraction as the output layer; adopting ReLu as the neuron type; adopting at least one optimization algorithm selected from Adam, SGD and FTRL; preferably FTRL. Preferably, the parameters of the TensorFlow learning system further include: the learning rate is set to 0.002; the number of hidden layers is 1; the number of neurons in the hidden layer is 200. Thereby, the result is accurate and reliable. It should be noted that the term "weight" used in the context is a relative concept for an indicator. The weight of an indicator refers to the relative importance of the indicator in the overall evaluation. For example, a certain "predetermined window weight" refers to the relative importance of the certain predetermined window among all predetermined windows. A certain "connection weight" refers to the relative importance of the connection between two different layers in all connections between two different layers.

Regarding the method for determining the fetal fraction, PCT/CN2018/07204 (title of invention: Method and device for determining proportion of free nucleic acid of predetermined origin in biological sample) has a detailed introduction, so that it will not be repeated here, and the full text of the patent application is incorporated here by reference. The method can be used to acquire fetal fraction data simply, quickly and accurately. At the same time, the acquired fetal fraction data can be more effectively applied to the method of the present invention to determine whether the fetus has chromosomal aneuploidy.

In addition, after acquiring the nucleic acid sequencing data from a pregnant woman sample, not only the fetal fraction can be determined, but also the estimated fraction by the predetermined chromosome can be further determined.

The term "the estimated fraction" as used in the context refers to a measure that characterizes a difference between the DNA content of a specific chromosome and the DNA content of a normal chromosome. Specifically, it can be denoted with a difference between the number of sequencing reads of a specific chromosome and the number of sequencing reads of normal chromosome. For example, in an ideal state, for chromosomes with trisomy, the estimated fraction is an amount that represents the DNA content of one extra chromosome, while for normal chromosomes, the estimated fraction is 0 because there is no extra chromosome.

Because this context focuses on the analysis of chromosomal aneuploidy, the term "normal chromosome" used in the context refers to a chromosome without chromosomal aneuploidy, which does not mean that the chromosome does not have other abnormalities.

In addition, the expression "number of sequencing reads of..." as mentioned several times in the context, such as "number of sequencing reads of normal chromosome", "number of sequencing reads of specific chromosome", "number of sequencing reads that falls into a predetermined pair" and so on, refers to the number of sequencing reads that can be matched with the region. For example, when a nucleic acid sequencing result is aligned with a reference sequence such as hg19 by using a conventional software such as SOAP, a sequencing read that can be mapped with a specific region is regarded as a sequencing read of the region. In addition, according to an embodiment of the present invention, it can also only select the "uniquely mapped sequencing read" as the sequencing read that falls into a specific region, that is, the sequencing read that can only be mapped to one position of the reference sequence. Furthermore, when considering tendentious bias caused by influence of certain factors such as GC content on the sequencing device during sequencing, the acquired number of sequencing reads may be corrected, for example, corrected by GC content. Specifically, for example, according to an embodiment of the present invention, the steps for determining the corrected number of sequencing reads comprise:

Dividing a reference sequence such as a human genome (GRCh37) into a plurality of windows, aligning the high-throughput sequencing reads with the human reference genome (GRCh37) by using bwa (0.7.7-r441), counting the information where the sequencing reads are mapped to each window of each chromosome, that is, the number of sequencing reads in each window, recording the number of sequencing reads in the i-th window as $UR_i$, and recording the GC content of the reference genome in the *i*-th window as $GC_i$; fitting the number of sequencing reads and the GC content for each window, and correcting the number of sequencing reads in the original window based on the fitting coefficient, and recording the effective sequence number after GC correction in the *i*-th window as $URA_i$.

Thereby, the accuracy and precision of the high-throughput sequencing data analysis can be effectively improved by selecting uniquely-mapped sequencing reads and performing GC content correction processing.

As mentioned earlier, "the estimated fraction" refers to a measure that characterizes a difference between the DNA content of a specific chromosome and the DNA content of normal chromosome. Therefore, the estimated fraction can be used as an important indicator for characterizing pregnant women samples. According to an embodiment of the present invention, the estimated fraction is determined based on a difference between the number of sequencing reads of a predetermined chromosome and the number of sequencing reads of a first comparison chromosome.

The term "predetermined chromosome" as used herein comprises a chromosome to be tested, that is, a chromosome for which aneuploidy needs to be determined. In addition, the predetermined chromosome also comprises a second comparison chromosome. According to an embodiment of the present invention, the second comparison chromosome comprises at least one autosome. It should be noted that the estimated fraction is calculated separately for each predetermined chromosome, so that for each of the chromosome to be tested and the second comparison chromosome, the estimated fraction corresponding to the chromosome will be obtained respectively. In addition, it should be noted that the first comparison chromosome and the second comparison chromosome as well as the chromosome to be tested are derived from the same sample, instead of using data from other samples for analysis.

According to an embodiment of the present invention, the second comparison chromosome comprises at least 10 autosomes. According to an embodiment of the present invention, the second comparison chromosome comprises 15 autosomes. In addition, as mentioned above, the estimated fraction can be used as an indicator to characterize whether a chromosome is abnormal. Therefore, the second comparison chromosome can be selected by using the estimated fraction. According to an embodiment of the present invention, it further comprises: determining the estimated fractions by a plurality of autosomes; and selecting target autosomes from the sorted autosomes as the second comparison chromosome according to a priority order from small to large. According to the above description, the smaller the estimated fraction, the higher the probability that the chromosome is a normal chromosome. For example, by sorting the estimated fractions by each all autosomes (the absolute values of the absolute estimated fractions can be used) from small to large, then the top 15 autosomes with smaller estimated fractions may be selected as the second comparison chromosomes. Therefore, under the premise of uncertain chromosomal aneuploidy status, a suitable autosome may be selected as the second comparison chromosome. Certainly, those skilled in the art would understand that, it may be determined through experience whether there is an abnormality in the number of chromosomes in practice. For example, it can be found through statistical analysis that some chromosomes have almost no aneuploidy, so that these chromosomes can be regarded as the second comparison chromosomes.

In addition, regarding the first comparison chromosome, as mentioned above, the estimated fraction is to characterize the difference between the characteristic chromosome and the normal chromosome. Therefore, according to an embodiment of the present invention, the first comparison chromosome comprises at least one autosome that is different from the predetermined chromosome. It should be noted that the first comparison chromosome and the second comparison chromosome as mentioned here may be overlapped. Specifically, when calculating the estimated fraction, a specific chromosome will be selected from the predetermined chromosomes, therefore, although the rest chromosomes may be covered by the meaning of "second comparison chromosome", they still belong to the concept of "autosomes different from the predetermined chromosome". For example, if chromosome 23 is selected as the chromosome to be tested and chromosomes 2 to 5 are used as the second comparison chromosomes, when the estimated fraction by chromosome 23 is calculated, chromosomes 2 to 5 can still be used as the first comparison chromosome. In addition, according to an embodiment of the present invention, the first comparison chromosome may comprise a plurality of autosomes, and the average number of sequencing reads thereof may be selected for calculating the estimated fraction. In this way, the efficiency and accuracy of sequencing data analysis can be further improved. According to an embodiment of the present invention, the number of sequencing reads of the first comparison chromosome is an average number of sequencing reads of a plurality of autosomes, and the plurality of autosomes comprises at least one autosome that is known to have no aneuploidy. According to an embodiment of the present invention, the number of sequencing reads of the first comparison chromosome is an average number of sequencing reads of at least 15 autosomes. Optionally, the number of sequencing reads of the first comparison chromosome is an average number of sequencing reads of at least 20 autosomes. Optionally, the number of sequencing reads of the first comparison chromosome is an average number of sequencing reads of all autosomes. In this way, by selecting an average number of sequencing reads for a plurality of chromosomes, a difference between chromosomes can be eliminated.

According to an embodiment of the present invention, the estimated fraction is determined according to the following formula:

$$F_j = 2 \times \left| R_j - R_r \right| / R_r$$

wherein,

$j$ represents the serial number of a chromosome the estimated fraction of which needs to be determined,

$F_j$ represents the estimated fraction by the chromosome $j$,

$R_r$ represents the average number of sequencing reads of the plurality of autosomes, and

$R_j$ represents the number of sequencing reads of the chromosome $j$.

The inventors found that the estimated fraction as calculated by this formula could be effectively applied to the subsequent machine learning classification model.

As mentioned above, the fetal fraction and the estimated fraction as determined in this step are both affected by chromosomal aneuploidy in different degrees. Therefore, these two parameters can be used in subsequent aneuploidy detection.

**S300: Determination of first feature and second feature**

After the fetal fraction and the estimated fraction are determined, these parameters can be further used as the feature values of the sample, so that the analysis may be performed by further using machine learning.

Specifically, according to an embodiment of the present invention, the first feature is determined by a difference between the estimated fraction by the chromosome to be tested and the estimated fraction by the second comparison chromosome, and the second feature is determined by a difference between the previously determined estimated fraction by the chromosome to be tested and fetal fraction. Thereby, the obtained first feature and second feature can be regarded as features that may be affected by aneuploidy, and thus can be effectively applied to subsequent analysis. According to an embodiment of the present invention, those skilled in the art can use a variety of algorithms to characterize the aforementioned differences, for example, by calculating value differences, value ratios, and so on.

As mentioned above, the estimated fraction by the second comparison chromosome is preferably an average estimated fraction by a plurality of autosomes. As a result, the efficiency and accuracy of analysis can be further improved.

In addition, according to an embodiment of the present invention, the first feature is determined by the following formula:

$$X_1 = F_i - F_r$$

wherein,

$X_1$ represents the first feature,

$i$ represents the serial number of the chromosome to be tested,

$F_i$ represents the estimated fraction by the chromosome to be tested,

$F_r$ represents the average value of the estimated fractions by the second comparison chromosomes.

According to an embodiment of the present invention, the second feature is determined by the following formula:

$$X_2 = \frac{F_i}{F_a}$$

wherein,

$X_2$ represents the second feature,
$i$ represents the serial number of the chromosome to be tested,
$F_i$ represents the estimated fraction by the chromosome to be tested,
$F_a$ represents the fetal fraction.

According to an embodiment of the present invention, the first feature and the second feature thus obtained can reflect the difference adopted by each, moreover the obtained values are all on the same order of magnitude, thereby avoiding the situation in which a single parameter excessively influences the analysis result. If the selection of features is inappropriate, the biases may appear in the subsequent analysis results. For example, in the K model, the distance between samples should be calculated according to the features of the samples (for example, if the features of sample $x_1$ is $\left(x_1^{(1)}, x_1^{(2)}\right)$, the features of sample $x_2$ is $\left(x_2^{(1)}, x_2^{(2)}\right)$, then the distance between the samples $x_1$ and $x_2$ is

$$d_{12} = \sqrt{(x_1^{(1)} - x_2^{(1)})^2 + (x_1^{(2)} - x_2^{(2)})^2}$$ ; if the feature values between the two samples are extremely different, for example, the distance is $d_{12} = \sqrt{(0.23 - 0.15)^2 + (2000 - 3000)^2}$ , it is obvious that the second dimensional feature will have a greater influence on the distance although the two-dimensional features are equally important.

In order to eliminate this influence, according to an embodiment of the present invention, the first feature and the second feature thus obtained are standardized before the subsequent steps, so that the absolute values of the first feature and the second feature are independently between 0 and 1. According to an embodiment of the present invention, the method for standardizing the first feature and the second feature is not particularly limited. Specifically, the following methods can be used to process a batch of data of the same dimension (all thereof are the first feature or the second feature) according to the following formula

$$newValue = (oldValue - min)/(max - min)$$

wherein, min and max are the minimum and maximum values of this batch of data, oldvale represents the value before processing, and newvalue represents the value after normalization processing.

As a result, it is possible to eliminate the excessive influence of a certain feature on the final analysis result and thus improve the accuracy of analysis result.

**S400: Determination of aneuploidy based on first feature and second feature**

As mentioned above, the values of the first feature and the second feature are both affected by aneuploidy. Therefore, after obtaining the first feature and the second feature, the corresponding data of a control sample are used to determine whether the fetus has an aneuploidy for the chromosome to be tested. Specifically, the control sample comprises a positive sample and a negative sample. The positive sample has an aneuploidy for the chromosome to be tested, and the negative sample does not have an aneuploidy for the chromosome to be tested.

By using the first feature and the second feature as classification features, and classifying the sample to be tested into the positive sample or the negative sample for the chromosome to be tested, the determination of whether the chromosome to be tested has an aneuploidy can be achieved. According to an embodiment of the present invention, the inventors found in the research process that the accuracy of analysis would be further improved when the number of positive samples and the number of negative samples are in a certain ratio. For example, according to an embodiment of the present invention, the ratio of the number of positive samples and the number of negative samples is not less than 1:4. According to an embodiment of the present invention, the ratio of the number of positive samples and the number of negative samples does not exceed 4:1. According to an embodiment of the present invention, the ratio of the number of positive samples and the number of negative samples is 1:0.1-5. According to an embodiment of the present invention, the ratio of the number of positive samples and the number of negative samples is 1:0.25~4. The inventors found that the bias of the model results can be avoided by using the above ratio. The inventors found that if there are too many positive samples, the result will be biased to be positive, that is, the false positive rate will be elevated, and if there are too many negative samples, the result will be biased to be negative, that is, the false negative rate will be elevated.

According to an embodiment of the present invention, neither the positive sample nor the negative sample has aneuploidy for chromosomes other than the chromosome to be tested. As a result, the classification reference ability of the control sample can be further improved.

According to an embodiment of the present invention, the method of using the first feature and the second feature for classification is not particularly limited, and various machine learning methods, such as neural network, SVM method, etc., can be used. During the in-depth research, the inventors found that the number of training sets required by the neural network is relatively large, and the SVM may require additional parameters for classification to improve the accuracy of classification. According to an embodiment of the present invention, the first feature and the second feature may be used to determine a two-dimensional feature vector of the pregnant woman sample and the control sample, a distance between the samples is determined by the two-dimensional feature vector, and the pregnant woman sample is classified as positive sample or negative sample so as to determine whether the fetus has an aneuploidy for the chromosome to be tested. According to an embodiment of the present invention, the distance used includes, but is not limited to, an Euclidean distance, a Manhattan distance, or a Chebyshev distance.

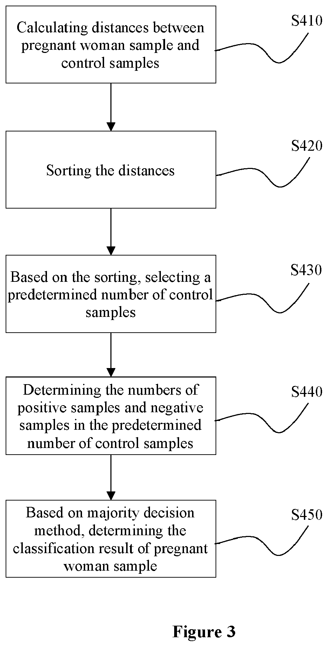

Specifically, according to an embodiment of the present invention, a k-nearest neighbor method (KNN) model can be used for classification analysis. For ease of understanding, the process of KNN model is briefly described as follows by referring to Figure 3:

According to an embodiment of the present invention, the classification processing comprises the following steps:

S410: calculating the distance between the pregnant woman sample and each of the control samples, respectively;

S420: sorting the obtained distances, wherein the sorting is based on an order from small to large;

S430: selecting a predetermined number of control samples from small to large based on the obtained sorting (this predetermined number is the $k$ value in the KNN model);

S440: determining the number of positive samples and the number of negative samples in the obtained predetermined number of control samples, respectively;

S450: determining the classification result of the pregnant woman sample based on a majority decision method.

According to an embodiment of the present invention, the predetermined number is not more than 20. According to an embodiment of the present invention, the predetermined number is 3 to 10. In order to facilitate processing, the $k$ value can be an odd number to avoid situations where a decision cannot be made. Of course, those skilled in the art can understand that the $k$ value finally selected for different chromosome to be tested may be different. For example, according to an embodiment of the present invention, the finally selected $k$ value for T13 and T18 detection is 7, and the finally selected $k$ value for T21 detection is 9.

In addition, according to an embodiment of the present invention, the distances between the sample to be tested and the predetermined control samples may be weighted in advance before the sorting. Thus, the accuracy of detection can be further improved.

Those skilled in the art can understand that the weighting coefficients of these weighting processes or the $k$ value of the KNN model can be obtained through machine learning by using known samples as training sets for training.

Specifically, according to an embodiment of the present invention, it can be performed through the following steps:

A. Selection of sample set

Samples with return visit results are selected as sample set, and divided into training set, test set and verification set in a ratio of 6:2:2.

B. Model training

Model input: $k$ value; training data set $T = \{(x_1,y_1), (x_2,y_2), ..., (x_N,y_N)\}$, in which $x_i \in R^n$ is the n-dimensional feature vector of sample; $y_i \in \{+1, -1\}$, $i = 1,2,..., N$ are the negative or positive labels of sample (-1 denotes negative, +1 denotes positive), and N is the size of sample set.

Model output: category y to which sample $x$ belongs.

C: Model verification

Initialization $k = 1$, $k$ value is continuously adjusted on the basis of validation set (methods such as cross-validation and grid-search may be used) until the predictive ability of the model shows good accuracy.

D: Model prediction

The trained model is used to make a prediction on the test set to evaluate the prediction performance of the model.

Thereby, this method can effectively determine whether the fetus has an aneuploidy for the chromosome to be tested. In addition, according to an embodiment of the present invention, in the process of implementing the method, it is found in this method that the strategy of setting threshold based on the number of sequencing reads in the prior art is replaced, the detection gray area is avoided, the sample detection cycle is also shortened in the method, and thus the customer experience is improved, and the sequencing and testing costs are significantly reduced.

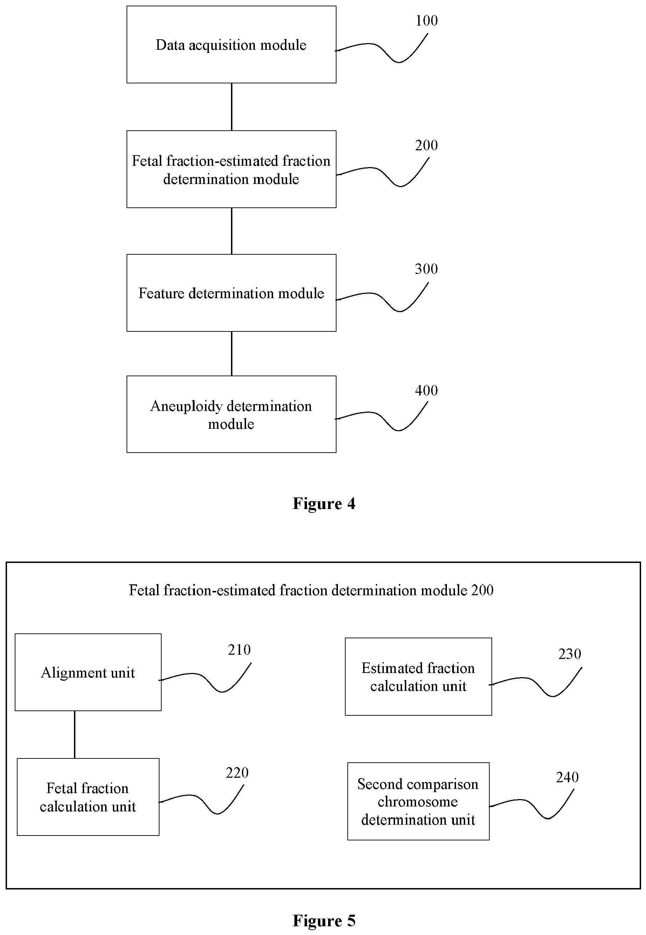

In the second aspect of the present invention, corresponding to the foregoing method, an embodiment of the present application also provides a corresponding device for implementing the foregoing method. Specifically, the present invention provides a device for determining whether a fetus has a chromosomal aneuploidy. Referring to Figure 4, the device for determining whether a fetus has a chromosomal aneuploidy comprises:

A data acquisition module 100, which is configured to acquire nucleic acid sequencing data from a pregnant woman sample, wherein the pregnant woman sample comprises a fetal free nucleic acid, and the nucleic acid sequencing data are composed of a plurality of sequencing reads;

A fetal fraction- estimated fraction determination module 200, which is configured to determine a fetal fraction of the pregnant woman sample and the estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome;

A feature determination module 300, which is configured to determine a first feature based on a difference between the estimated fraction by the chromosome to be tested and the estimated fraction by the second comparison chromosome, and to determine a second feature based on a difference between the estimated fraction by the chromosome to be tested and the fetal fraction; and

A aneuploidy determination module 400, which is configured to determine whether the fetus of the pregnant woman has an aneuploidy for the chromosome to be tested based on the first feature and the second feature by using corresponding data of control sample, wherein the control sample comprises a positive sample and a negative sample, the positive sample has an aneuploidy for the chromosome to be tested, and the negative sample does not have an aneuploidy for the chromosome to be tested.

By using the device for determining whether a fetus has a chromosomal aneuploidy according to an embodiment of the present invention, the forgoing method for determining whether a fetus has a chromosomal aneuploidy can be effectively implemented, thereby it can be effectively determined whether the fetus has an aneuploidy for the chromosome to be tested. In addition, according to an embodiment of the present invention, in the process of implementing the method of the present invention, it is found in this method that the strategy of setting threshold based on the number of sequencing reads in the prior art is replaced, the detection gray area is avoided, the sample detection cycle is also shortened, and thus the customer experience is improved, and the sequencing and testing costs are significantly reduced.

Referring to Figure 5, according to an embodiment of the present invention, the fetal fraction- estimated fraction determination module 200 comprises:

A comparison unit 210, which is configured to compare the nucleic acid sequencing data from the pregnant woman sample with a reference sequence, so as to determine the number of sequencing reads that fall into a predetermined window; and

A fetal fraction calculation unit 220, which is configured to determine the fetal fraction of the pregnant woman sample based on the number of sequencing reads that fall into the predetermined window.

According to an embodiment of the present invention, the fetal fraction- estimated fraction determination module 200 further comprises:

the estimated fraction calculation unit 230, which is configured to determine the estimated fraction according to the following formula:

$$F_j = 2 \times |R_j - R_r|/R_r$$

wherein,

$j$ represents the serial number of a chromosome the estimated fraction of which needs to be determined,

$F_j$ represents the estimated fraction by the chromosome $j$,

$R_r$ represents the average number of sequencing reads of the plurality of autosomes, and

$R_j$ represents the number of sequencing reads of the chromosome $j$.

According to an embodiment of the present invention, the fetal fraction- estimated fraction determination module 200 comprises:

A second comparison chromosome determination unit 240, which is configured to sort the estimated fractions by a plurality of autosomes in a priority order from small to large, and select target autosomes from the sorted autosomes as the second comparison chromosome.

According to an embodiment of the present invention, the feature determination module 300 comprises:
A first feature determination unit 310, which is configured to determine the first feature by the following formula:

$$X_1 = F_i - F_r$$

wherein,

$X_1$ represents the first feature,
$i$ represents the serial number of the chromosome to be tested,
$F_i$ represents the estimated fraction by the chromosome to be tested,
$F_r$ represents the average value of the estimated fraction by the second comparison chromosome.

According to an embodiment of the present invention, the feature determination module 300 further comprises:
A second feature determination unit 320, which is configured to determine the second feature by the following formula:

$$X_2 = \frac{F_i}{F_a}$$

wherein,

$X_2$ represents the second feature,
$i$ represents the serial number of the chromosome to be tested,
$F_i$ represents the estimated fraction by the chromosome to be tested,
$F_a$ represents the fetal fraction.

According to an embodiment of the present invention, the feature determination module 300 further comprises:
A standardization processing unit 330, which is configured to perform standardization processing on the first feature and the second feature, so that the absolute values of the first feature and the second feature are independently between 0 to 1.

According to an embodiment of the present invention, the aneuploidy determination module 400 is configured to determine a two-dimensional feature vector of the pregnant woman sample and the control samples, determine a distance between samples based on the two-dimensional feature vector, and classify the pregnant woman sample as positive sample or negative sample, so as to determine whether the fetus has an aneuploidy for the chromosome to be tested.

According to an embodiment of the present invention, the distance is an Euclidean distance, a Manhattan distance or a Chebyshev distance.

According to an embodiment of the present invention, the aneuploidy determination module is configured to determine a classification result of the pregnant woman sample by using a $k$-nearest neighbor model.

According to an embodiment of the present invention, the $k$-nearest neighbor model adopts a $k$ value of not exceeding 20.

According to an embodiment of the present invention, the $k$-nearest neighbor model adopts a $k$ value of 3 to 10.

According to an embodiment of the present invention, in the $k$-nearest neighbor model, the distance between samples is weighted.

It should be noted that the characteristics and advantages described above for the method for determining whether a fetus has a chromosome aneuploidy are all applicable to the device for determining whether a fetus has a chromosome aneuploidy, and thus will not be repeated here.

In the third aspect of the present invention, the present invention provides a computer-readable storage medium, on which a computer program is stored, characterized in that, when the program is executed by a processor, the steps of the aforementioned method for determining whether a fetus has a chromosomal aneuploidy are implemented. Therefore, the aforementioned method for determining whether a fetus has a chromosomal aneuploidy can be effectively implemented, so that it can be effectively determined whether the fetus has an aneuploidy for the chromosome to be tested. In addition, according to an embodiment of the present invention, in the process of implementing the method, it is found in the method that the strategy of setting threshold based on the number of sequencing reads in the prior art is replaced, the detection gray area is avoided, the sample detection cycle is also shortened, and thus the customer experience is improved, and the sequencing and testing costs are significantly reduced.

Those skilled in the art can understand that the characteristics and advantages described above for the method for determining whether a fetus has a chromosomal aneuploidy are applicable to the computer-readable storage medium, and thus will not be repeated here.

In the fourth aspect of the present invention, the present invention provides an electronic device, which comprises: the aforementioned computer-readable storage medium; and one or more processors, which are configured to execute the program stored on the computer-readable storage medium. Therefore, the aforementioned method for determining whether a fetus has a chromosomal aneuploidy can be effectively implemented, so that it can be effectively determined whether the fetus has an aneuploidy for the chromosome to be tested. In addition, according to an embodiment of the present invention, in the process of implementing the method, it is found in the method that the strategy of setting threshold based on the number of sequencing reads in the prior art is replaced, the detection gray area is avoided, the sample detection cycle is also shortened, and thus the customer experience is improved, and the sequencing and testing costs are significantly reduced. Those skilled in the art can understand that the characteristics and advantages described above for the method for determining whether a fetus has a chromosomal aneuploidy are applicable to the electronic device, and thus will not be repeated here.

In the fifth aspect of the present invention, the present invention provides a method for constructing a machine learning classification model. According to an embodiment of the present invention, the method comprises:

(a) performing the following steps for each of a plurality of pregnant women samples:

Acquiring nucleic acid sequencing data from the pregnant woman sample, wherein the pregnant woman sample comprise a fetal free nucleic acid, the nucleic acid sequencing data are composed of a plurality of sequencing reads, the pregnant woman sample comprises at least one positive sample and at least one negative sample, the positive sample has an aneuploidy for a chromosome to be tested, and the negative sample does not have an aneuploidy for the chromosome to be tested;

Determining a fetal fraction of the pregnant woman sample and the estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; and determining a first feature based on a difference between the estimated fraction by the chromosome to be tested and the estimated fraction by the second comparison chromosome, and determining a second feature based on a difference between the estimated fraction by the chromosome to be tested and the fetal fraction;

(b) performing a machine learning training by taking the plurality of pregnant women samples as samples and using the first features and the second features of the samples, so as to construct a machine learning classification model for determining whether the fetus has an aneuploidy.

By using this method, according to an embodiment of the present invention, a machine learning classification model can be effectively constructed, so that the classification model can be further used to identify and classify unknown samples to determine whether there is a chromosomal aneuploidy for a specific chromosome. According to an embodiment of the present invention, the machine learning classification model is a KNN model. According to an embodiment of the present invention, the KNN model adopts an Euclidean distance.

Those skilled in the art can understand that the characteristics and advantages described above for the method for determining whether a fetus has a chromosomal aneuploidy are applicable to the method for constructing model, and thus will not be repeated here.

In the sixth aspect of the present invention, the present invention provides a device for constructing a machine learning classification model.

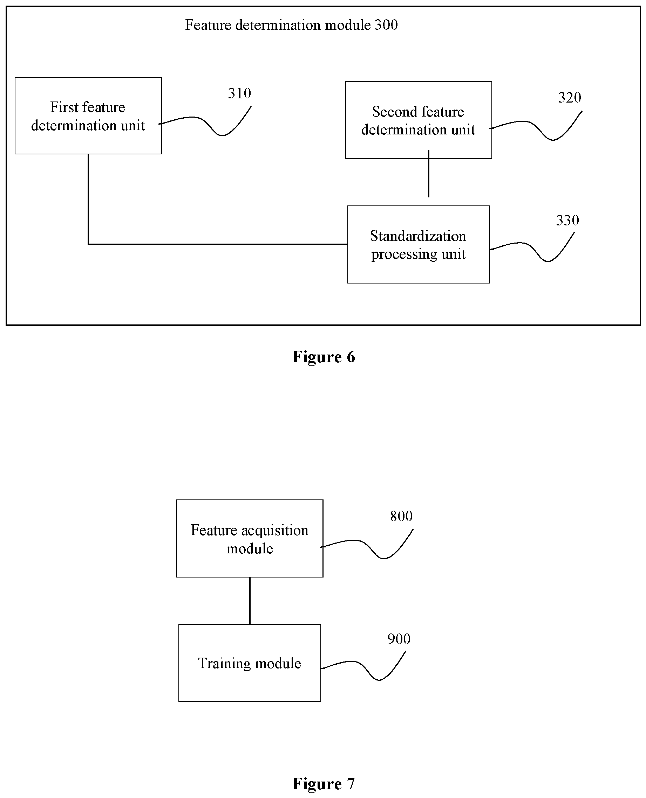

Referring to Figure 7, the device comprises:

A feature acquisition module 800, which is configured to perform the following steps for each of a plurality of pregnant women samples: acquiring nucleic acid sequencing data from the pregnant woman sample, wherein the pregnant woman sample comprise a fetal free nucleic acid, the nucleic acid sequencing data are composed of a plurality of sequencing reads, the pregnant woman sample comprises at least one positive sample and at least one negative sample, the positive sample has an aneuploidy for a chromosome to be tested, and the negative sample does not have an aneuploidy for the chromosome to be tested; determining a fetal fraction of the pregnant woman sample and the estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; and determining a first feature based on a difference between the estimated fraction by the chromosome to be tested and the estimated fraction by the second comparison chromosome, and determining a second feature based on a difference between the estimated fraction by the chromosome to be tested and the fetal fraction;

A training model 900, which is configured to perform a machine learning training by taking the plurality of pregnant women samples as samples, so as to construct a machine learning classification model for determining whether the fetus has an aneuploidy. By using this device, the aforementioned method for constructing a machine learning classification model can be effectively implemented, thereby effectively constructing a machine learning classification model, so that the classification model can be further used to identify and classify unknown samples to determine whether there is a chromosomal aneuploidy for a specific chromosome.

According to an embodiment of the present invention, the machine learning classification model is a KNN model.

By using this device, according to an embodiment of the present invention, a machine learning classification model can be effectively constructed, so that the classification model can be further used to identify and classify unknown samples to determine whether there is a chromosomal aneuploidy for a specific chromosome. According to an embodiment of the present invention, the machine learning classification model is a KNN model. According to an embodiment of the present invention, the KNN model adopts an Euclidean distance.

Those skilled in the art can understand that the characteristics and advantages described above for the method for determining whether a fetus has a chromosomal aneuploidy are applicable to the device for constructing model, and thus will not be repeated here.

In the seventh aspect of the present invention, the present invention provides a computer-readable storage medium, on which a computer program is stored, and when the program is executed by a processor, the steps for constructing a machine learning classification method described in the preceding claims are implemented. As a result, the aforementioned method for constructing a machine learning classification model can be effectively implemented, thereby effectively constructing a machine learning classification model, so that the classification model can be further used to identify and classify unknown samples to determine whether there is a chromosomal aneuploidy for a specific chromosome. Those skilled in the art can understand that the characteristics and advantages described above for the method for determining whether a fetus has a chromosomal aneuploidy are applicable to the computer-readable storage medium for constructing model, and thus will not be repeated here.

The technical solution of the present invention will be explained below in conjunction with examples. Those skilled in the art will understand that the following examples are only used to illustrate the present invention and should not be regarded as limiting the scope of the present invention. Where specific techniques or conditions are not indicated in the examples, the procedures shall be carried out in accordance with the techniques or conditions described in the literature in the art or in accordance with the product specification. If specific conditions are not indicated in the examples, it shall be carried out in accordance with conventional conditions or conditions recommended by the manufacturers. The reagents or instruments used which manufacturers are not given are all conventional products that can be obtained on the market.

**Example 1:**

In this example, model training and model prediction were performed on the basis of 3,075 samples with return visit results (including male fetus: 1716 cases, female fetus: 1359 cases, negative samples: 2215 cases, chromosome 21 trisomy (T21): 637 cases, trisomy 18 (T18): 165 cases, trisomy 13 (T13): 58 cases) from 2017 to 2018 based on BGISEQ-500 platform.

First, the reference genome (GRCh37) was classified into adjacent windows according to a fixed length (60K was used in this method), the windows in the N area were filtered out, and the GC contents in the windows were counted, thereby obtaining the reference window file hg19.gc;

Next, the sequence (35bp) after SE sequencing based on the CG platform was aligned (BWA V0.7.7-r441) with the reference genome (GRCh37);

Filtering and preliminary statistics: According to the comparison results, the uniquely completely mapped sequences were selected and repetitive sequences and sequences with base mismatches were removed to obtain effective sequences, and then the number and GC content of effective sequences in each window were counted according to the window in the hg19.gc file;
GC correction, of which steps were as follows:

For a certain sample, the number of effective sequences in the i-th window was recorded as $UR_i$, the GC content of the reference genome in this window was recorded as $GC_i$ (recorded in the hg19.gc file), the mean of numbers of effective sequences in all windows for autosomes (chromosomes 1-22) was recorded as $\overline{UR}$;

The numbers of effective sequences and GC contents in all windows for autosome were used to perform fitting (cubic spline fitting was used in this example), thereby obtaining their relationship: $ur = f(gc)$;

Correction for windows of all chromosomes: $UR_i \times (\overline{UR}/f(GC_i))$, $i = 1,2,3, ..., N$; the number of effective sequences in the $i$-th window after GC correction was recorded as $URA_i$.

The estimated fraction by each chromosome was calculated according to the following formula:
the formula for calculating the estimated fraction was as follows:

$$F_j = \begin{cases} 2 \times \dfrac{\overline{URAA_j} - \overline{URAA}}{\overline{URAA}}, & j = 1, ..., 22 \\[3mm] 2 \times \dfrac{\overline{URAA} - \overline{URAA_j}}{\overline{URAA}}, & j = X \end{cases}$$

$j$ represented the serial number of the chromosome, $\overline{URAA_j}$ represented the number of GC-corrected sequencing reads that were matched with the reference sequence of chromosome $j$, and $\overline{URAA}$ represented the average number of GC-corrected sequencing reads that were matched with all autosomal reference sequences.

The fetal fraction was determined according to the conventional method or the method disclosed in PCT/CN2018/072045.

The KNN model training and sample prediction were performed based on the sample set, and the specific steps were as follows:

(a) Sample set division and data preprocessing: The sample set was randomly divided into training set, validation set and test set at a ratio of 6:2:2; data preprocessing was performed on the samples of the training set, validation set and test set, respectively, so that each sample got a two-dimensional feature vector and a corresponding label (-1 denoted negative, +1 denoted positive).

(b) Selection of hyperparameter $k$: It was found by the inventors that if a smaller value of $k$ was selected, which was equivalent to using a training sample set of samples in a smaller neighborhood for prediction, the prediction result would be very sensitive to adjacent sample points, and the overall model would become complicated and prone to overfitting; if a larger value of $k$ was selected, which was equivalent to using a the training sample set in a larger neighborhood for prediction, the training sample set far from (not similar with) the newly input samples at this time would also influence the prediction result and make a prediction wrong; and in a limiting case that with $k$ of a certain value, no matter what category the newly input sample belonged to, it would be simply predicted to belong to the class with the most in the training sample set. Therefore, in the practice of the present invention, a relatively small value was generally adopted for $k$.

(c) Model training: including two parts: KNN model training and $k$ value selection. At this time, a Euclidean distance and a majority voting rule were selected.

KNN model training: for classification decision function:

$$f: R^n \to \{c_1, c_2\} = \{-1, +1\}$$

Wherein $x \in R^n$ was n-dimensional feature space, -1 and +1 were sample labels (-1 denoted negative, +1 denoted positive) respectively. Then, the probability of misclassification was:

$$P(Y \neq f(X)) = 1 - P(Y = f(X))$$

For a given sample $x \in X$, the set constituted of the nearest $k$ neighbor training sample points was $N_k(x)$. If the region covered $N_k(x)$ was classified as $c_j$, then the probability of misclassification was:

$$\frac{1}{k}\sum_{x_i \in N_k(x)} I\big(y_i \neq c_j\big) = 1 - \frac{1}{k}\sum_{x_i \in N_k(x)} I\big(y_i = c_j\big)$$

In order to minimize the probability of misclassification, $\frac{1}{k}\sum_{x_i \in N_k(x)} I(y_i = c_j)$ must be maximized. Therefore, after selecting the $k$ value, the process of model training was the process of maximizing $\frac{1}{k}\sum_{x_i \in N_k(x)} I(y_i = c_j)$.

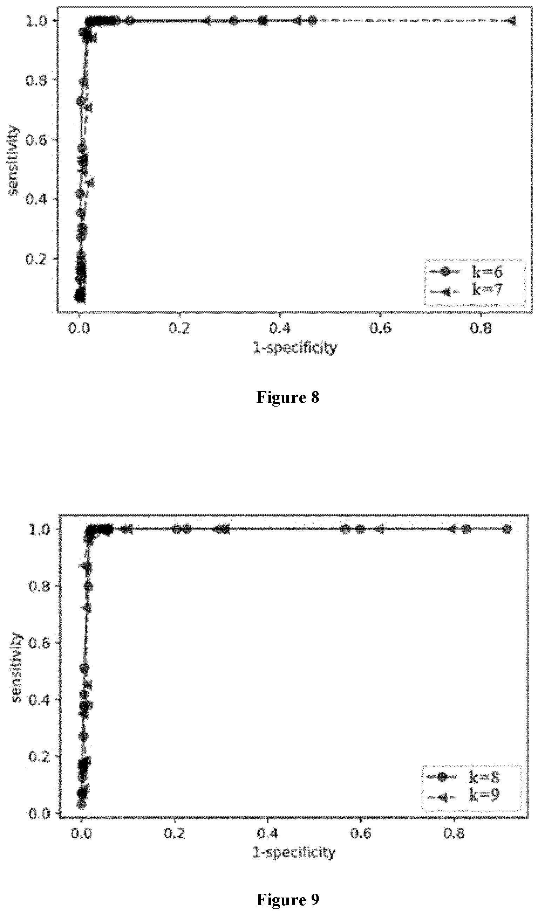

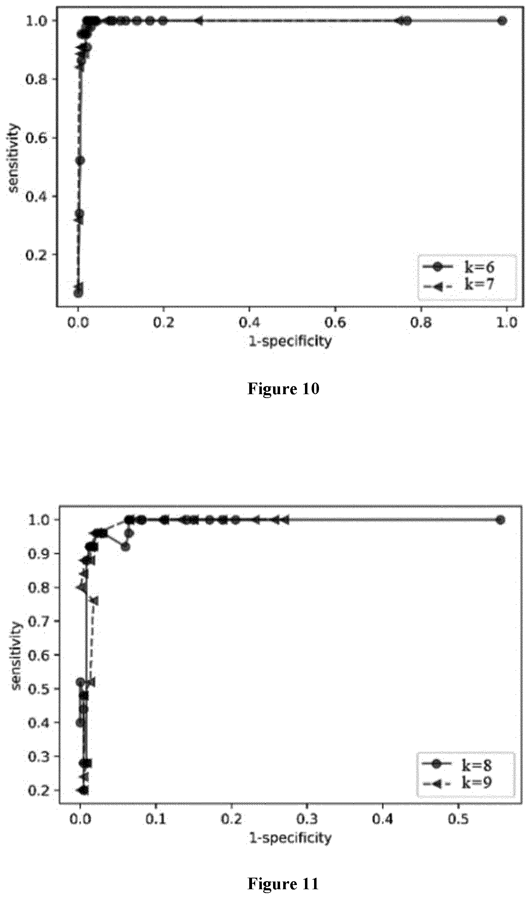

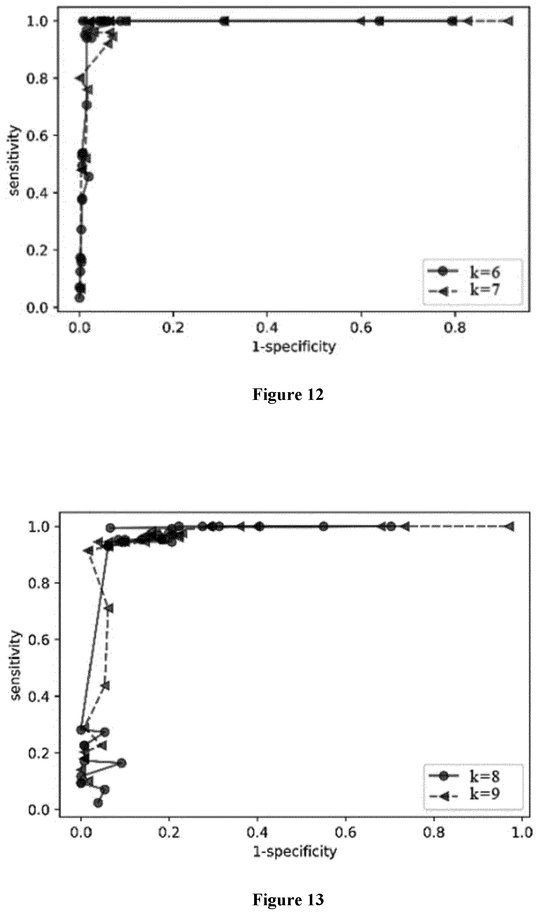

Selection of $k$ value: Initialization $k = 1$ ($k \in \{1,2, ...,20\}$) was carried out, the $k$ value was determined on the basis of validation set using a linear search method. The results were shown in Figures 8-~13, in which Figures 8~13 were all ROC curve diagrams, which respectively represented the corresponding ROC curve diagrams when different values were selected for parameter k, which reflected the effect of corresponding classifiers, and the evaluation standard was AUC that referred to an area under ROC curve, and the larger the AUC, the better the classification performance. Figures 8 and 9 showed the ROC curves when the KNN model was used to detect T21 with the parameter k of 6, 7, 8 and 9, respectively. Figures 10 and 11 showed the ROC curves when the KNN model was used to detect T18 with the parameter k of 6, 7, 8 and 9, respectively. Figures 12 and 13 showed the ROC curves when the KNN model was used to detect T13 with the parameter k of 6, 7, 8 and 9, respectively. According to the results of Figures 8 to 13, the finally selected k for T13 and T18 was 7, and the finally selected k for T21 was 9.

(d) Model prediction: Based on the model trained in the above steps, the predication for test set was carried out, and the prediction results were shown in the following tables.

| T21 | | Karyotype results | |
|---|---|---|---|
| | | Size of positive samples | Size of negative samples |
| KNN model results | Size of positive samples | 122 | 3 |
| | Size of negative samples | 0 | 483 |

| T18 | | Karyotype results | |
|---|---|---|---|
| | | Size of positive samples | Size of negative samples |
| KNN model results | Size of positive samples | 33 | 5 |
| | Size of negative samples | 0 | 570 |

| T13 | | Karyotype results | |
|---|---|---|---|
| | | Size of positive samples | Size of negative samples |
| KNN model results | Size of positive samples | 10 | 6 |
| | Size of negative samples | 0 | 592 |

The sensitivity, specificity, PPV and ACC of the detection were calculated and the results were shown in the below tables.

| | Sensitivity | Specificity | PPV | ACC |
|---|---|---|---|---|
| T21 | 100% | 99.38% | 97.60% | 99.51% |
| T18 | 100% | 99.13% | 86.84% | 99.18% |
| T13 | 100% | 99.00% | 62.50% | 99.01% |

2.5 Comparison with SVM model

Based on the same training set, validation set and test set, SVM (Support Vector Machine) method was used to classify the negative and positive samples. The results were as follows:

| T21 | | Karyotype results | |
|---|---|---|---|
| | | Size of positive samples | Size of negative samples |
| SVM model results | Size of positive samples | 122 | 14 |
| | Size of negative samples | 0 | 472 |

| T18 | | Karyotype results | |
|---|---|---|---|
| | | Size of positive samples | Size of negative samples |
| SVM model results | Size of positive samples | 33 | 8 |
| | Size of negative samples | 0 | 567 |

| T13 | | Karyotype results | |
|---|---|---|---|
| | | Size of positive samples | Size of negative samples |
| SVM model results | Size of positive samples | 10 | 8 |
| | Size of negative samples | 0 | 590 |

The sensitivity, specificity, PPV and ACC of the detection were calculated and the results were shown in the below tables.

| | Sensitivity | Specificity | PPV | ACC |
|---|---|---|---|---|
| T21 | 100% | 97.13% | 89.71% | 97.71% |
| T18 | 100% | 98.61% | 80.49% | 98.69% |
| T13 | 100% | 98.67% | 55.56% | 98.69% |

It could be seen from the data that either the KNN model or the SVM model showed no undetected error in the detection of T13, T18 and T21 for the test set, and had sensitivity of 100%. However, in the T21 detection, the SVM model had 14 false positive samples, while the KNN model had only 3 false positive samples; in the T18 detection, the SVM model had 8 false positive samples, while the KNN model had only 5 false positive samples; in the T13 test, the SVM model had 8 false positive samples, while the KNN model had 6 false positive samples. Regardless of T21, T18 or T13, the KNN model always had a lower false positive rate than the SVM model.

According to the analysis of the inventors, the main reason for the lower false positive rate of the KNN model than the SVM model is: the model itself, that is, the KNN is mainly based on clustering and has a lot of refined clusters, while the SVM has only two simple categories and thus is inferior to the KNN in the level of detail.

In the description, the descriptions about terms "an embodiment", "some embodiments", "examples", "specific examples", or "some examples" etc. mean that the specific characteristics, structures, materials or features described in conjunction with the embodiment or example are included in at least one embodiment or example of the present invention. In the description, the schematic representations of the above terms do not necessarily refer to the same embodiment or example. Moreover, the specific characteristics, structures, materials or features as described may be combined in any one or more embodiments or examples in a suitable manner.

Although the examples of the present invention have been shown and described, those of ordinary skill in the art can understand that various changes, modifications, substitutions and modifications may be made to these examples without departing from the principle and purpose of the present invention. The scope of the present invention is defined by the claims and their equivalents.

**Claims**

1. A method for determining whether a fetus has a chromosomal aneuploidy, **characterized by** comprising:

(1) acquiring nucleic acid sequencing data from a pregnant woman sample, wherein the pregnant woman sample comprises a fetal free nucleic acid, and the nucleic acid sequencing data are composed of a plurality of sequencing reads;

(2) determining a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome;

(3) determining a first feature based on a difference between the estimated fraction by the chromosome to be tested and the estimated fraction by the second comparison chromosome, and determining a second feature based on a difference between the estimated fraction by the chromosome to be tested and the fetal fraction; and

(4) determining whether the fetus has an aneuploidy for the chromosome to be tested based on the first feature and the second feature by using corresponding data of a control sample, wherein the control sample comprises a positive sample and a negative sample, the positive sample has an aneuploidy for the chromosome to be tested, and the negative sample does not have an aneuploidy for the chromosome to be tested.

2. The method according to claim 1, **characterized in that** the pregnant woman sample comprises peripheral blood of a pregnant woman.

3. The method according to claim 1, **characterized in that** the nucleic acid sequencing data are obtained by paired-end sequencing, single-end sequencing, or single-molecule sequencing.

4. The method according to claim 1, **characterized in that** the fetal fraction is determined by the following steps:

(a) comparing the nucleic acid sequencing data from the pregnant woman sample with a reference sequence, so as to determine the number of sequencing reads that fall into a predetermined window; and
(b) determining the fetal fraction of the pregnant woman sample based on the number of sequencing reads that fall into the predetermined window.

5. The method according to claim 1, **characterized in that** in the step (2), the number of sequencing reads of the first comparison chromosome is an average number of sequencing reads of a plurality of autosomes, and the plurality of autosomes comprise at least one autosome that is known to have no aneuploidy.

6. The method according to claim 5, **characterized in that** in the step (2), the number of sequencing reads of the first comparison chromosome is an average number of sequencing reads of at least 15 autosomes,

optionally, the number of sequencing reads of the first comparison chromosome is an average number of sequencing reads of at least 20 autosomes,
optionally, the number of sequencing reads of the first comparison chromosome is an average number of sequencing reads of all autosomes.

7. The method according to claim 5, **characterized in that** the estimated fraction is determined according to the following formula:

$$F_j = 2 \times \left| R_j - R_r \right| / R_r$$

wherein,

$j$ represents the serial number of a chromosome the estimated fraction of which needs to be determined,
$F_j$ represents the estimated fraction by the chromosome $j$,
$R_r$ represents the average number of sequencing reads of the plurality of autosomes, and
$R_j$ represents the number of sequencing reads of the chromosome $j$.

8. The method according to claim 1, **characterized in that** in the step (2), the second comparison chromosome comprises a plurality of autosomes having no aneuploidy, and in the step (3), the first feature is determined based on a difference between the estimated fraction by the chromosome to be tested and an average value of the estimated fraction by the second comparison chromosome.

9. The method according to claim 8, **characterized in that** the second comparison chromosome comprises at least 10 autosomes.

10. The method according to claim 8, **characterized in that** the second comparison chromosome comprises 15 autosomes.

11. The method according to claim 8, **characterized by** further comprising:

   determining the estimated fractions by a plurality of autosomes; and
   selecting target autosomes from the sorted autosomes as the second comparison chromosome in a priority order from small to large.

12. The method according to claim 1, **characterized in that** the first feature is determined by the following formula:

$$X_1 = F_i - F_r$$

wherein,

   $X_1$ represents the first feature,
   $i$ represents the serial number of the chromosome to be tested,
   $F_i$ represents the estimated fraction by the chromosome to be tested,
   $F_r$ represents the average value of the estimated fraction by the second comparison chromosome.

13. The method according to claim 12, **characterized in that** the second feature is determined by the following formula:

$$X_2 = \frac{F_i}{F_a}$$

wherein,

   $X_2$ represents the second feature,
   $i$ represents the serial number of the chromosome to be tested,
   $F_i$ represents the estimated fraction by the chromosome to be tested,
   $F_a$ represents the fetal fraction.

14. The method according to any one of claims 1 to 13, **characterized in that**, before performing the step (4), the first feature and the second feature are standardized, so that the absolute values of the first feature and the second feature are independently between 0 to 1.

15. The method according to claim 1, **characterized in that**, in the step (4), the numbers of the positive sample and the negative sample have a ratio of not less than 1:4.

16. The method according to claim 1, **characterized in that** in the step (4), the numbers of the positive sample and the negative sample have a ratio of not exceeding 4:1.

17. The method according to claim 1, **characterized in that** in the step (4), the numbers of the positive sample and the negative sample have a ratio of 1:0.1-5.

18. The method according to claim 1, **characterized in that** in the step (4), the numbers of the positive sample and the negative sample have a ratio of 1:0.25~4.

19. The method according to claim 1, **characterized in that** neither the positive sample nor the negative sample has aneuploidy for chromosomes other than the chromosome to be tested.

20. The method according to claim 1, **characterized in that** in the step (4), a two-dimensional feature vector of the pregnant woman sample and the control samples is determined based on the first feature and the second feature, a distance between samples is determined based on the two-dimensional feature vector, and the pregnant woman sample is classified as positive sample or negative sample, so as to determine whether the fetus has an aneuploidy for the chromosome to be tested.

21. The method according to claim 20, **characterized in that** the distance is an Euclidean distance, a Manhattan distance or a Chebyshev distance.

22. The method according to claim 20, **characterized in that** the step (4) further comprises:

   (4-1) calculating distances between the pregnant woman sample and the control samples respectively;
   (4-2) sorting the obtained distances, the sorting being based on the order from small to large;
   (4-3) selecting a predetermined number of control samples in order from small to large based on the sorting;
   (4-4) determining the number of positive samples and the number of negative samples in the predetermined number of control samples respectively;
   (4-5) determining a classification result of the pregnant woman sample based on a majority decision method.

23. The method according to claim 22, **characterized in that** the predetermined number does not exceed 20.

24. The method according to claim 22, **characterized in that** the predetermined number is 3 to 10.

25. The method according to claim 22, **characterized in that**, in the step (4-2), the distances between the sample to be tested and the predetermined control samples are weighted in advance before the sorting is performed.

26. A device for determining whether a fetus has a chromosomal aneuploidy, **characterized by** comprising:

   a data acquisition module, which is configured to acquire nucleic acid sequencing data from a pregnant woman sample, wherein the pregnant woman sample comprises a fetal free nucleic acid, and the nucleic acid sequencing data are composed of a plurality of sequencing reads;
   a fetal fraction- estimated fraction determination module, which is configured to determine a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome;
   a feature determination module, which is configured to determine a first feature based on a difference between the estimated fraction by the chromosome to be tested and the estimated fraction by the second comparison chromosome, and to determine a second feature based on a difference between the estimated fraction by the chromosome to be tested and the fetal fraction; and
   an aneuploidy determination module, which is configured to determine whether the fetus of the pregnant woman has an aneuploidy for the chromosome to be tested based on the first feature and the second feature by using corresponding data of control sample, wherein the control sample comprises a positive sample and a negative sample, the positive sample has an aneuploidy for the chromosome to be tested, and the negative sample does not have an aneuploidy for the chromosome to be tested.

27. The device according to claim 26, **characterized in that** the fetal fraction- estimated fraction determination module comprises:

   an alignment unit, which is configured to align the nucleic acid sequencing data from the pregnant woman sample with a reference sequence, so as to determine the number of sequencing reads that fall into a predetermined window; and
   a fetal fraction calculation unit, which is configured to determine the fetal fraction of the pregnant woman sample based on the number of sequencing reads that fall into the predetermined window.

**28.** The device according to claim 26, **characterized in that** the fetal fraction- estimated fraction determination module comprises:
an estimated fraction calculation unit, which is configured to determine the estimated fraction according to the following formula:

$$F_j = 2 \times \left| R_j - R_r \right| / R_r$$

wherein,

$j$ represents the serial number of a chromosome the estimated fraction of which needs to be determined,
$F_j$ represents the estimated fraction by the chromosome $j$,
$R_r$ represents the average number of sequencing reads of the plurality of autosomes, and
$R_j$ represents the number of sequencing reads of the chromosome $j$.

**29.** The device according to claim 26, **characterized in that** the fetal fraction- estimated fraction determination module comprises:
a second comparison chromosome determination unit, which is configured to sort the estimated fractions by a plurality of autosomes in a priority order from small to large, and select target autosomes from the sorted autosomes as the second comparison chromosome.

**30.** The device according to claim 26, **characterized in that** the feature determination module comprises:
a first feature determination unit, which is configured to determine the first feature by the following formula:

$$X_1 = F_i - F_r$$

wherein,

$X_1$ represents the first feature,
$i$ represents the serial number of the chromosome to be tested,
$F_i$ represents the estimated fraction by the chromosome to be tested,
$F_r$ represents the average value of the estimated fraction by the second comparison chromosome.

**31.** The device according to claim 26, **characterized in that** the feature determination module comprises:
a second feature determination unit, which is configured to determine the second feature by the following formula:

$$X_2 = \frac{F_i}{F_a}$$

wherein,

$X_2$ represents the second feature,
$i$ represents the serial number of the chromosome to be tested,
$F_i$ represents the estimated fraction by the chromosome to be tested,
$F_a$ represents the fetal fraction.

**32.** The device according to claim 26, **characterized in that** the feature determination module comprises:
a standardization processing unit, which is configured to perform standardization processing on the first feature and the second feature, so that the absolute values of the first feature and the second feature are independently between 0 to 1.

**33.** The device according to claim 26, **characterized in that** the aneuploidy determination module is configured to determine a two-dimensional feature vector of the pregnant woman sample and the control samples, to determine a distance between samples based on the two-dimensional feature vector, and to classify the pregnant woman sample as positive sample or negative sample, so as to determine whether the fetus has an aneuploidy for the chromosome to be tested.

34. The device according to claim 33, **characterized in that** the distance is an Euclidean distance, a Manhattan distance or a Chebyshev distance.

35. The device according to claim 26, **characterized in that** the aneuploidy determination module is configured to determine a classification result of the pregnant woman sample by using a *k*-nearest neighbor model.

36. The device according to claim 35, **characterized in that** the *k*-nearest neighbor model adopts a *k* value of not exceeding 20.

37. The device according to claim 35, **characterized in that** the *k*-nearest neighbor model adopts a *k* value of 3 to 10.

38. The device according to claim 35, **characterized in that** in the *k*-nearest neighbor model, the distance between samples is weighted.

39. A computer-readable storage medium, on which a computer program is stored, **characterized in that**, when the program is executed by a processor, the steps of the method according to any one of claims 1-25 are implemented.

40. An electronic device, **characterized by** comprising:

the computer-readable storage medium according to claim 39; and
one or more processors, which are configured to execute the program stored on the computer-readable storage medium.

41. A method for constructing a machine learning classification model, **characterized by** comprising:

(a) performing the following steps for each of a plurality of pregnant women samples:

acquiring nucleic acid sequencing data from the pregnant woman sample, wherein the pregnant woman sample comprise a fetal free nucleic acid, the nucleic acid sequencing data are composed of a plurality of sequencing reads, the pregnant woman sample comprises at least one positive sample and at least one negative sample, the positive sample has an aneuploidy for a chromosome to be tested, and the negative sample does not have an aneuploidy for the chromosome to be tested;
determining a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; and
determining a first feature based on a difference between the estimated fraction by the chromosome to be tested and the estimated fraction by the second comparison chromosome, and determining a second feature based on a difference between the estimated fraction by the chromosome to be tested and the fetal fraction;

(b) performing a machine learning training by taking the plurality of pregnant women samples as samples and using the first features and the second features of the samples, so as to construct a machine learning classification model for determining whether the fetus has an aneuploidy.

42. The method according to claim 41, **characterized in that** the machine learning classification model is a KNN model.

43. The method according to claim 42, **characterized in that** the KNN model adopts a Euclidean distance.

44. A device for constructing a machine learning classification model, **characterized by** comprising:

a feature acquisition module, which is configured to perform the following steps for each of a plurality of pregnant women samples:

acquiring nucleic acid sequencing data from the pregnant woman sample, wherein the pregnant woman sample comprise a fetal free nucleic acid, the nucleic acid sequencing data are composed of a plurality of sequencing reads, the pregnant woman sample comprises at least one positive sample and at least one negative sample, the positive sample has an aneuploidy for a chromosome to be tested, and the negative sample does not have an aneuploidy for the chromosome to be tested;

determining a fetal fraction of the pregnant woman sample and an estimated fraction by a predetermined chromosome based on the nucleic acid sequencing data, wherein the estimated fraction by a predetermined chromosome is determined based on a difference between a number of sequencing reads of the predetermined chromosome and a number of sequencing reads of a first comparison chromosome, the predetermined chromosome comprises a chromosome to be tested and a second comparison chromosome, and the first comparison chromosome comprises at least one autosome different from the predetermined chromosome; and

determining a second feature based on a difference between the estimated fraction by the chromosome to be tested and the fetal fraction, and determining a first feature based on a difference between the estimated fraction by the chromosome to be tested and the estimated fraction by the second comparison chromosome;

a training model, which is configured to perform a machine learning training by taking the plurality of pregnant women samples as samples, so as to construct a machine learning classification model for determining whether the fetus has an aneuploidy.

**45.** The device according to claim 44, **characterized in that** the machine learning classification model is a KNN model.

**46.** A computer-readable storage medium, on which a computer program is stored, **characterized in that** when the program is executed by a processor, the steps of the method according to any one of claims 41 to 43 are implemented.

```
┌─────────────────────────┐
│   Acquisition of nucleic │        S100
│  acid sequencing data    │────────╮  ╱
│  from pregnant women     │         ╲╱
│        sample            │
└─────────────────────────┘
            │
            ▼
┌─────────────────────────┐
│ Determination of the     │        S200
│ estimated fraction by    │────────╮  ╱
│ the chromosome to be     │         ╲╱
│ tested and fetal fraction│
└─────────────────────────┘
            │
            ▼
┌─────────────────────────┐
│ Determination of first   │        S300
│ feature and second       │────────╮  ╱
│ feature                  │         ╲╱
└─────────────────────────┘
            │
            ▼
┌─────────────────────────┐
│ Determination of         │        S400
│ aneuploidy based on      │────────╮  ╱
│ first feature and second │         ╲╱
│ feature                  │
└─────────────────────────┘
```

**Figure 1**

```
┌─────────────────────────┐
│ Determining the number   │        S210
│ of sequencing reads that │────────╮  ╱
│ fall into a predetermined│         ╲╱
│ window                   │
└─────────────────────────┘
            │
            ▼
┌─────────────────────────┐
│ Determining fetal        │        S220
│ fraction based on the    │────────╮  ╱
│ number of sequencing     │         ╲╱
│ reads that fall into the │
│ predetermined window     │
└─────────────────────────┘
```

**Figure 2**

```
┌─────────────────────────┐
│  Calculating distances  │          S410
│ between pregnant woman   │
│  sample and control      │
│       samples            │
└─────────────────────────┘
            │
            ▼
┌─────────────────────────┐
│                         │          S420
│  Sorting the distances  │
│                         │
└─────────────────────────┘
            │
            ▼
┌─────────────────────────┐
│ Based on the sorting,   │          S430
│ selecting a predetermined│
│ number of control samples│
└─────────────────────────┘
            │
            ▼
┌─────────────────────────┐
│ Determining the numbers │          S440
│ of positive samples and │
│ negative samples in the │
│ predetermined number of │
│     control samples     │
└─────────────────────────┘
            │
            ▼
┌─────────────────────────┐
│ Based on majority       │          S450
│ decision method,        │
│ determining the         │
│ classification result of│
│  pregnant woman sample  │
└─────────────────────────┘
```

**Figure 3**

Figure 4

Figure 5

Feature determination module 300

First feature
determination unit

310

Second feature
determination unit

320

Standardization
processing unit

330

**Figure 6**

Feature acquisition
module

800

Training module

900

**Figure 7**

**Figure 8**

**Figure 9**

**Figure 10**

**Figure 11**

**Figure 12**

**Figure 13**

## INTERNATIONAL SEARCH REPORT

| International application No. |
|---|
| **PCT/CN2019/130625** |

### A. CLASSIFICATION OF SUBJECT MATTER

C12Q 1/68(2018.01)i; G16B 20/10(2019.01)i; G16B 25/10(2019.01)i

According to International Patent Classification (IPC) or to both national classification and IPC

### B. FIELDS SEARCHED

Minimum documentation searched (classification system followed by classification symbols)

C12Q;G16B

Documentation searched other than minimum documentation to the extent that such documents are included in the fields searched

Electronic data base consulted during the international search (name of data base and, where practicable, search terms used)

DWPI, VEN, SIPOABS, CNABS, CNTXT, USTXT, EPTXT, WOTXT, 万方数据库, CNKI, PubMed, ISI web of Knowledge, GenBank, EBI, STN: 染色体, 反估浓度, 非整倍性, 胎儿, 非整倍, 深圳华大, 孕妇, 血浆, 无创, 第一, 第二, 预定, 比较, 特征, 比率, 平均, 估计, 核酸测序, 数据, 数量, 读段数目, 张红云, 柴相花, 周丽君, 袁玉英, 王梦杰, chromosomal aneuploidy, positive, negative, fetal, noninvasive, prenatal, device, computer

### C. DOCUMENTS CONSIDERED TO BE RELEVANT

| Category* | Citation of document, with indication, where appropriate, of the relevant passages | Relevant to claim No. |
|---|---|---|
| A | WO 2014153755 A1 (BGI SHENZHEN) 02 October 2014 (2014-10-02)<br>entire document | 1-46 |
| A | WO 2015006932 A1 (BGI SHENZHEN CO., LIMITED) 22 January 2015 (2015-01-22)<br>entire document | 1-46 |
| A | WO 2015089726 A1 (BGI SHENZHEN CO., LIMITED) 25 June 2015 (2015-06-25)<br>entire document | 1-46 |
| A | WO 2018132400 A1 (QUEST DIAGNOSTICS INVESTMENTS LLC) 19 July 2018 (2018-07-19)<br>entire document | 1-46 |
| A | WO 2011130880 A1 (BGI SHENZHEN CO., LIMITED) 27 October 2011 (2011-10-27)<br>entire document | 1-46 |
| A | CN 104789686 A (ANNOROAD GENE TECHNOLOGY (BEIJING) CO., LTD.) 22 July 2015 (2015-07-22)<br>entire document | 1-46 |

☑ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 September 2020** | **27 September 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

EP 4 086 356 A1

**INTERNATIONAL SEARCH REPORT**

| International application No. |
| --- |
| **PCT/CN2019/130625** |

C.    DOCUMENTS CONSIDERED TO BE RELEVANT

| Category* | Citation of document, with indication, where appropriate, of the relevant passages | Relevant to claim No. |
| --- | --- | --- |
| A | WO 2013040773 A1 (BGI SHENZHEN CO., LIMITED et al.) 28 March 2013 (2013-03-28) entire document | 1-46 |
| A | CN 106520940 A (BGI-SHENZHEN) 22 March 2017 (2017-03-22) entire document | 1-46 |
| A | CN 104789466 A (ANNOROAD GENE TECHNOLOGY (BEIJING) CO., LTD.) 22 July 2015 (2015-07-22) entire document | 1-46 |
| A | CN 104232777 A (BGI TIANJIN CO., LTD. et al.) 24 December 2014 (2014-12-24) entire document | 1-46 |
| A | CHIU, R.W.K. et al. "Noninvasive prenatal diagnosis of fetal chromosomal aneuploidy by massively parallel genomic sequencing of DNA in maternal plasma." *PNAS.*, Vol. 105, No. 51, 23 December 2008 (2008-12-23), pp. 20458-20463 | 1-46 |
| A | LAU, T.K. et al. "Noninvasive prenatal diagnosis of common fetal chromosomal aneuploidies by maternal plasma DNA sequencing." *Journal of Maternal-Fetal and Neonatal Medicine.*, 31 December 2012 (2012-12-31), pp. 1-5 | 1-46 |

Form PCT/ISA/210 (second sheet) (January 2015)

36

International application No.

**PCT/CN2019/130625**

| Patent document cited in search report | | | Publication date (day/month/year) | Patent family member(s) | | | Publication date (day/month/year) |
|---|---|---|---|---|---|---|---|
| WO | 2014153755 | A1 | 02 October 2014 | CN | 104205106 | A | 10 December 2014 |
| WO | 2015006932 | A1 | 22 January 2015 | US | 2016154931 | A1 | 02 June 2016 |
| | | | | EP | 3023504 | A1 | 25 May 2016 |
| | | | | HK | 1208888 | A1 | 18 March 2016 |
| | | | | EP | 3023504 | A4 | 05 April 2017 |
| | | | | CN | 104520437 | A | 15 April 2015 |
| | | | | CN | 104520437 | B | 14 September 2016 |
| | | | | EP | 3023504 | B1 | 02 October 2019 |
| WO | 2015089726 | A1 | 25 June 2015 | CN | 105765076 | A | 13 July 2016 |
| | | | | CN | 105765076 | B | 19 July 2019 |
| WO | 2018132400 | A1 | 19 July 2018 | BR | 112019014208 | A2 | 17 March 2020 |
| | | | | MX | 2019008320 | A | 09 September 2019 |
| | | | | EP | 3568472 | A1 | 20 November 2019 |
| | | | | CN | 110770341 | A | 07 February 2020 |
| | | | | CA | 3049442 | A1 | 19 July 2018 |
| WO | 2011130880 | A1 | 27 October 2011 | CN | 102753703 | A | 24 October 2012 |
| | | | | CN | 102753703 | B | 24 December 2014 |
| CN | 104789686 | A | 22 July 2015 | CN | 104789686 | B | 07 September 2018 |
| WO | 2013040773 | A1 | 28 March 2013 | JP | 2014527822 | A | 23 October 2014 |
| | | | | CN | 103764841 | A | 30 April 2014 |
| | | | | CN | 103764841 | B | 29 June 2016 |
| | | | | JP | 5964432 | B2 | 03 August 2016 |
| | | | | TW | 201313895 | A | 01 April 2013 |
| | | | | HK | 1192284 | A1 | 10 March 2017 |
| | | | | TW | I534262 | B | 21 May 2016 |
| | | | | US | 2014228226 | A1 | 14 August 2014 |
| CN | 106520940 | A | 22 March 2017 | | None | | |
| CN | 104789466 | A | 22 July 2015 | CN | 104789466 | B | 13 March 2018 |
| CN | 104232777 | A | 24 December 2014 | HK | 1201886 | A1 | 11 September 2015 |
| | | | | CN | 104232777 | B | 24 August 2016 |

Form PCT/ISA/210 (patent family annex) (January 2015)

**REFERENCES CITED IN THE DESCRIPTION**

**Patent documents cited in the description**

- CN 201807204 W

- CN 2018072045 W